# EUROPÄISCHE PATENTSCHRIFT

(11) **EP 3 589 347 B1**
(45) Veröffentlichungstag und Bekanntmachung des Hinweises auf die Patenterteilung: **19.02.2025**
(21) Anmeldenummer: 18709517.9
(22) Anmeldetag: 01.03.2018
(51) Int. Cl.: A61M 16/10, A61M 16/20, F16L 5/04, A62C 2/06, F16K 17/38, A61M 16/06, A61M 16/08, A62C 2/04

(54) **VORRICHTUNG ZUM BRANDSCHUTZ BEI DER THERAPIE MIT SAUERSTOFFANGEREICHERTEN ATEMGASEN**
APPARATUS FOR FIRE PROTECTION DURING THERAPY BY MEANS OF OXYGEN-ENRICHED RESPIRATORY GASES
DISPOSITIF DE PROTECTION CONTRE LES BRÛLURES LORS DE LA THÉRAPIE AVEC DES GAZ RESPIRATOIRES ENRICHIS EN OXYGÈNE

(30) Priorität: 02.03.2017 DE 102017001925; 15.03.2017 DE 102017002544
(43) Veröffentlichungstag der Anmeldung: 08.01.2020
(73) Patentinhaber: Haindl, Hans, Dr., 30974 Wennigsen (DE)
(72) Erfinder: Haindl, Hans, Dr., 30974 Wennigsen (DE)
(74) Vertreter: Vossius & Partner Patentanwälte Rechtsanwälte mbB
(86) Internationale Anmeldenummer: PCT/EP2018/055107
(87) Internationale Veröffentlichungsnummer: WO 2018/158396

(56) Entgegenhaltungen:
- EP-A2- 2 682 149
- WO-A1-2012/001951
- DE-U1- 202015 007 512
- GB-A- 2 504 553
- US-A- 5 927 312
- US-B1- 6 269 811

## Beschreibung

Die vorliegende Erfindung betrifft eine Vorrichtung zur Versorgung eines Patienten mit sauerstoffangereichertem Atemgas mit einer Brandschutzeinrichtung.

Patienten mit ausgeprägten Lungenfunktionsstörungen sind in ihrer Aktivität stark eingeschränkt, da sie häufig schon nach leichten körperlichen Belastungen Atemnot entwickeln. Die Lungenfunktionsstörungen können auch so ausgeprägt sein, dass die Patienten in Ruhe Atemnot verspüren. Sofern die Lungenfunktionsstörung nicht medikamentös zu lindern ist, z. B. durch Medikamente, die die Bronchien erweitern, bleibt nur die Möglichkeit, den Patienten mit einem erhöhten Sauerstoffanteil in der Atemluft zu versorgen, der dann auch bei eingeschränkter Lungenfunktion zu einer höheren Sauerstoffsättigung im Blut führt. Dadurch können die Patienten mit Hilfe von tragbaren Sauerstoffgeräten wieder aktiver werden oder, sofern sie dazu nicht in der Lage sind, kann zumindest die Atemnot gelindert werden.

Eine solche Versorgung mit Sauerstoff erfolgt in der Regel mit einem Schlauchsystem, mit dem einem Patienten Sauerstoff oder sauerstoffangereicherte Luft zugeführt werden kann. Der Sauerstoff kann bereitgestellt werden aus Druckflaschen, Flüssigsauerstoffgefäßen (Dewar), aus Sauerstoffkonzentratoren oder -generatoren oder aus zentralen Sauerstoffversorgungsanlagen. Der Sauerstoff kann dem Patienten über eine sogenannte Nasenbrille, über eine Atemmaske (geschlossen oder offen) oder über einen Beatmungstubus durch Mund / Nase oder Tracheostoma zugeführt werden.

Zahlreiche der sauerstoffbedürftigen Patienten leiden an einer sogenannten COPD, eine chronisch obstruktive Lungenerkrankung, die in der Regel durch langjähriges Rauchen ausgelöst wird. Ca. 20% der Patienten können es nicht unterlassen, trotz entsprechender Warnungen auch während der Sauerstoffbehandlung zu rauchen (vgl. West GA, Primeau P. Nonmedical hazards of long-term oxygen therapy. Respir Care. 1983; 28:906-12). Durch den Sauerstoff kommt es zu einer erheblichen Brandbeschleunigung, die Zigaretten können in Flammen aufgehen, dabei entzündet sich dann häufig auch die Kunststoff-Nasenbrille oder Atemmaske sowie die Kleidung oder die Bettwäsche des Patienten. Durch den nachströmenden Sauerstoff wird das Feuer erheblich beschleunigt und binnen kurzer Zeit kann das Bett des Patienten oder er selbst in hellen Flammen stehen. Todesfälle durch diese Brände sind berichtet worden (vgl. Morbidity and mortality weekly report. Fatal fires associated with smoking during long-term oxygen therapy. Maine, Massachussetts, New Hampshire, and Oklahoma. 2000-2007. MMWR. 2008; 57:852-854).

Es sind bereits Vorrichtungen bekannt, in denen der Gasfluss bei Druckabfall im System unterbunden wird. So offenbart beispielsweise US 6,269,811 B1 ein Druckunterstützungssystem, das auf eine Unterbrechung des Gasflusses abzielt, sobald ein primäres, druckerzeugendes System ausfällt. DE 29 29 163 A1 hingegen beschreibt ein Bedarfsatemventil, das an einer Gesichtsmaske angebracht ist und unter besonderen Bedingungen, beispielsweise wenn ein Druckabfall durch das Abnehmen der Maske vom Gesicht entsteht, schließen kann, um unnötige Gasverluste zu vermeiden. Diese Vorrichtungen bieten jedoch keine Lösung für das Problem der Flussunterbrechung bei Feuer.

Es gibt bereits verschiedene Systeme, den nachfließenden Sauerstoff zu stoppen und damit die Ausbreitung des Feuers zu verhindern oder zumindest zu vermindern. So beschreibt beispielsweise die WO 2014/020334 A1 ein Ventil, das in den Zuleitungsschlauch eingebaut werden kann und das, wenn es von den Flammen erreicht wird, den Zustrom automatisch stoppt, so dass kein Sauerstoff mehr nachfließen kann. Dieses System hat allerdings den Nachteil, dass es in der Regel in das Ende des Sauerstoffschlauches eingebaut wird, wo dieser sich in zwei Schläuche aufteilt, die dann die sogenannte Nasenbrille bilden, über die der Patient den Sauerstoff bekommt. Das Feuer muss sich also erst vom primären Brandherd über diese beiden Schläuche bis zum Anschluss an den gemeinsamen Zuleitungsschlauch ausbreiten, um das Ventil zu aktivieren. Dadurch fließt unnötig lang Sauerstoff nach. Außerdem kann auch, da sich der Brand auch über die Bettwäsche und/oder Kleidung des Patienten ausbreitet, der Zuleitungsschlauch vor dem Ventil von den Flammen ergriffen werden, schmelzen und dadurch der Sauerstoffzustrom wieder freigegeben werden.

EP 2 682 149 A2 beschreibt ein System, bei dem im Schlauch ein dünner Kupferdraht integriert ist, an dem eine Spannung liegt oder dessen Widerstand gemessen wird. Kommt es jetzt zum Entflammen des Schlauches, schmilzt dieser Kupferdraht durch und über ein entsprechendes Überwachungsgerät wird die Sauerstoffzufuhr unterbrochen. Diese Anordnung kann sicherlich deutlich früher als die in WO 2014/020334 A1 beschriebene den Zufluss von Sauerstoff unterbinden, sie ist aber sehr aufwendig in der Herstellung. Da ein geschlossener Stromkreis vorliegen muss, muss die Zuleitung zur Nasenbrille des Patienten über zwei Schläuche erfolgen, zwischen denen zuverlässige elektrische Kontakte bestehen müssen. Auch die elektrische Verbindung des Kupferdrahtes mit dem Überwachungsgerät ist aufwendig.

DE 20 2012 009 706 U1 beschreibt ein entsprechendes System mit einem Lichtleiter an Stelle des Kupferdrahtes, was noch aufwändiger sein dürfte.

US 5,927,312 beschreibt ein System mit einem sich selbst löschenden Schlauch, bei dem im Falle eines Brandes ein Verschluss ausgelöst wird, der den Gasstrom im Wesentlichen unterbinden soll. Dies wird durch eine Kombination verschiedener Materialien realisiert, wobei sich ein hitzeempfindliches Material ausdehnt, das an der gewünschten Verschlussstelle außen um die Leitung herum angebracht ist, bzw. zusammenzieht oder schmilzt, sofern es im Inneren der Leitung angebracht ist. Wie in US 5,927,312 jedoch bereits erwähnt, würde das Anbringen dieses Systems in den Nasenzuführungen zu einer nicht erwünschten Zunahme von Material und Gewicht im Gesichtsbereich führen. Im Übrigen hat das beschriebene System die gleichen Nachteile wie WO 2014/020334 A1.

DE 20 2015 007 512 U1 beschreibt einen Atemluftschlauch, der mit nicht brennbarem Material beschichtet ist und sich daher nicht ohne weiteres entzünden lässt. Dieser Atemluftschlauch verhindert aber nicht den Nachstrom von Sauerstoff und kann das Entflammen der Zigarette und die daraus folgende sauerstoffbeschleunigte Entzündung von Kleidung und Bettwäsche nicht verhindern.

Es ist demnach eine Aufgabe der vorliegenden Erfindung, ein technisch einfaches und in der Herstellung preisgünstiges System bereitzustellen, das einen frühen und sicheren Stopp des nachströmenden Sauerstoffes gewährleistet.

Diese Aufgabe wird mittels einer Vorrichtung zur Versorgung eines Patienten mit sauerstoffangereichertem Atemgas gemäß Anspruch 1 sowie durch ein Verfahren zum Betrieb einer solchen Vorrichtung nach Anspruch 14 gelöst. Bevorzugte Ausführungsformen der erfindungsgemäßen Vorrichtung und des erfindungsgemäßen Verfahrens werden in den abhängigen Ansprüchen beschrieben.

Dementsprechend richtet sich die vorliegende Erfindung auf eine Vorrichtung zur Versorgung eines Patienten mit sauerstoffangereichertem Atemgas. Die Vorrichtung weist eine Einrichtung zur Bereitstellung von sauerstoffangereichertem Atemgas, eine mit der Einrichtung verbundene Zuführleitung, über die das sauerstoffangereicherte Atemgas von der Einrichtung an den Patienten zugeführt werden kann, und einen Detektor auf, der dazu geeignet ist, eine Zunahme des Volumenstroms von sauerstoffangereichertem Atemgas und/oder einen Druckabfall des sauerstoffangereicherten Atemgases zu detektieren. Die Vorrichtung ist dazu geeignet und dazu eingerichtet, die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases zu unterbinden bzw. unterbrechen. Dabei weist die Zuführleitung einen patientennahen Bereich mit einem vergrößerten Strömungswiderstand auf, der derart beschaffen ist, dass der Strömungswiderstand unter Einwirkung von Hitze durch Schmelzen des Schlauches reduziert wird. Die Vorrichtung weist ferner ein Ventil auf, das die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases unterbindet, wobei das Ventil dazu geeignet ist, mechanisch selbsttätig die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases zu unterbinden.

Mit anderen Worten liegt der Erfindung das Prinzip zugrunde, dass direkt am Abgabeort des Sauerstoffs bzw. der sauerstoffangereicherten Atemluft eine Flussrestriktion erfolgt (im einfachsten Falle beispielsweise durch eine Querschnittsverengung der Zuführleitung), was zu einer relativen Druckerhöhung im proximalen Bereich der Zuführleitung führt. Bei einem Feuer im Gesichtsbereich des Patienten, z.B. durch eine sich entflammende Zigarette, wird diese Flussrestriktion wieder aufgehoben, da der Bereich mit einem vergrößerten Strömungswiderstand derart beschaffen ist, dass der Strömungswiderstand unter Einwirkung von Hitze reduziert wird. So kann beispielsweise die Querschnittsverengung unter Einwirkung von Hitze aufschmelzen. Dies bewirkt eine Zunahme des Volumenstroms bzw. einen Druckabfall in der Zuführleitung, was wiederum dazu führt, dass die Zufuhr des sauerstoffangereicherten Atemgases unterbunden wird. Dies erfolgt dadurch, dass das Ventil geschlossen wird.

Die erfindungsgemäße Vorrichtung ist technisch relativ einfach und daher kostengünstig in der Herstellung und garantiert einen frühzeitigen und sicheren Stopp der Sauerstoffzufuhr, da die Detektion des Feuers (über den Bereich mit einem vergrößerten Strömungswiderstand) patientennah erfolgt.

Bei der Einrichtung zur Bereitstellung von sauerstoffangereichertem Atemgas kann es sich um eine Druckflasche, ein Flüssigsauerstoffgefäß (Dewar), einen Sauerstoffkonzentrator oder -generator oder eine zentrale Sauerstoffversorgungsanlage handeln. Die Einrichtung ist dazu geeignet, ein Atemgas mit gegenüber Umgebungsluft erhöhtem Sauerstoffanteil bereitzustellen. Gegebenenfalls kann das sauerstoffangereicherte Atemgas auch reiner Sauerstoff sein. Je nach Art und Funktionsweise der Einrichtung zur Bereitstellung von sauerstoffangereichertem Atemgas bietet sich für die Unterbindung bzw. Unterbrechung der Atemgaszufuhr bevorzugt ein Abschalten (z.B. Konzentrator) oder ein Schließen eines Ventils (z.B. Druckflasche) an. Ersteres ließe sich auch sehr einfach als Vorschaltgerät realisieren, in das der Stecker beispielsweise des Konzentrators eingesteckt wird. Dies hat den Vorteil, dass bereits existierende Konzentratoren ohne kompliziertes oder teures Nachrüsten mit der erfindungsgemäßen Vorrichtung verwendet werden können.

Die Zuführleitung, über die das sauerstoffangereicherte Atemgas von der Einrichtung zur Bereitstellung von sauerstoffangereichertem Atemgas einem Patienten zugeführt werden kann, ist mit der Einrichtung zur Bereitstellung von sauerstoffangereichertem Atemgas direkt oder indirekt verbunden. Insbesondere kann eine Einrichtung zur Regelung des Zuführstroms und/oder des Drucks in der Zuführleitung vorhanden sein. Die Zuführleitung weist bevorzugt einen Zuführschlauch mit einer Nasenbrille oder einem Konnektor zur Verbindung mit einer Atemmaske oder einem Beatmungstubus auf. Bevorzugt ist der Bereich mit einem vergrößerten Strömungswiderstand am patientenseitigen Ende des Zuführschlauchs, an der Nasenbrille und/oder am Konnektor vorgesehen. Bevorzugt ist der Bereich mit einem vergrößerten Strömungswiderstand möglichst nah am Austrittsort des sauerstoffangereicherten Atemgases vorgesehen, da sich an diesem Austrittsort der Brand am schnellsten entwickelt. Bevorzugt ist der Abstand zwischen dem Bereich mit einem vergrößerten Strömungswiderstand und dem Austrittsort des sauerstoffangereicherten Atemgases kleiner als 10 cm, stärker bevorzugt kleiner als 5 cm und besonders bevorzugt kleiner als 3 cm.

Gemäß einer ersten bevorzugten Variante, die nicht unter den Wortlaut der Ansprüche fällt, wird die Zunahme des Volumenstroms von sauerstoffangereichertem Atemgas und/oder der Druckabfall des sauerstoffangereicherten Atemgases in der Zuführleitung detektiert bzw. gemessen und die Einrichtung zur Bereitstellung von sauerstoffangereichertem Atemgas bzw. das Ventil mittels einer geeigneten Steuerung abgeschaltet bzw. geschlossen. Hierfür weist die Vorrichtung bevorzugt einen Detektor auf, der dazu geeignet ist, eine Zunahme des Volumenstroms von sauerstoffangereichertem Atemgas in der Zuführleitung und/oder einen Druckabfall des sauerstoffangereicherten Atemgases in der Zuführleitung zu detektieren. Der Detektor kann in der Zuführleitung oder an der Einrichtung zur Bereitstellung von sauerstoffangereichertem Atemgas vorgesehen sein. Die Vorrichtung weist ferner bevorzugt eine Steuerung auf, die dazu geeignet ist, das Ventil zu schließen und/oder die Einrichtung zur Bereitstellung von sauerstoffangereichertem Atemgas abzuschalten, falls der Detektor eine Zunahme des Volumenstroms von sauerstoffangereichertem Atemgas und/oder einen Druckabfall des sauerstoffangereicherten Atemgases detektiert. Bevorzugt ist der Detektor dazu geeignet, eine relative Zunahme des Volumenstroms von sauerstoffangereichertem Atemgas in der Zuführleitung um mindestens 50%, bevorzugt um mindestens 30%, stärker bevorzugt um mindestens 20%, besonders bevorzugt um mindestens 10% zu detektieren. Alternativ oder zusätzlich ist der Detektor bevorzugt dazu geeignet, einen relativen Druckabfall des sauerstoffangereicherten Atemgases in der Zuführleitung von mindestens 50%, bevorzugt von 30%, stärker bevorzugt von mindestens 20% und besonders bevorzugt von mindestens 10% zu detektieren.

Bevorzugt ist die Steuerung dazu eingerichtet, im Normalbetrieb, bevorzugt nach einer vorbestimmten Zeitspanne nach dem Beginn der Bereitstellung von sauerstoffangereichertem Atemgas, mittels des Detektors den Volumenstrom von sauerstoffangereichertem Atemgas und/oder den Druck des sauerstoffangereicherten Atemgases zu messen und einen Schwellwert zu definieren. Die Steuerung ist ferner bevorzugt dazu eingerichtet, die Zufuhr des sauerstoffangereicherten Atemgases zu unterbinden, wenn eine prozentuale Mindestabweichung des gemessenen Volumenstroms von sauerstoffangereichertem Atemgas und/oder des gemessenen Drucks des sauerstoffangereicherten Atemgases vom Schwellwert ermittelt wird. Die prozentuale Mindestabweichung beträgt bevorzugt 50%, stärker bevorzugt 30%, noch stärker bevorzugt 20% und besonders bevorzugt 10%. Die vorbestimmte Zeitspanne beträgt bevorzugt mindestens 10 s, stärker bevorzugt mindestens 20 s, noch stärker bevorzugt mindestens 30 s und besonders bevorzugt mindestens 1 min. Dadurch wird sichergestellt, dass der Schwellwert erst dann bestimmt wird, wenn sich bereits ein Strömungsgleichgewicht eingestellt hat und idealerweise die Nasenbrille bereits am Patienten befestigt ist, so dass nachfolgend Abweichungen von diesem Normal- oder Schwellwert bestimmt werden können.

Zusätzlich oder alternativ kann die Vorrichtung eine Einrichtung bzw. einen Mechanismus aufweisen, der dafür sorgt, dass die Atemgaszufuhr kurz unterbrochen wird, wenn die Zuführleitung mit der Einrichtung zur Bereitstellung von sauerstoffangereichertem Atemgas verbunden wird. Wenn der Anwender beispielsweise erst den Konzentrator anwirft und dann erst den die Zuführleitung an den Konzentrator anschließt, dann kann die Steuerung der Vorrichtung einen falschen Schwellwert einstellen. Durch die kurze Unterbrechung wird die Steuerung getriggert, einen neuen Schwellwert nach dem Verbinden der Zuführleitung mit dem Konzentrator zu bestimmen. Hierfür könnte beispielsweise der Schlauchanschluss der Zuführleitung axial geringgradig verschieblich sein, so dass beim Aufstecken und Abnehmen des Schlauches jeweils die Atemgaszufuhr unterbrochen wird oder die Fluss- bzw. Druckmessung neu ausgelöst wird.

Gemäß einer zweiten, erfindungsgemäßen Variante ist das Ventil selbst dazu geeignet, mechanisch selbsttätig die Zufuhr des sauerstoffangereicherten Atemgases im Falle einer Zunahme des Volumenstroms von sauerstoffangereichertem Atemgas durch das Ventil und/oder eines Druckabfalls des sauerstoffangereicherten Atemgases am Ventil zu unterbinden. Bevorzugt weist das Ventil eine Feder oder einen Magneten auf, die/der das Ventil entgegen der Stromrichtung des sauerstoffangereicherten Atemgases in die Offenstellung vorspannt. Bevorzugt ist dabei die Federkonstante der Feder bzw. die Kraft des Magneten derart bemessen, dass das Ventil bei einem Volumenstrom von 5 l/min und einem schlagartigen Druckabfall von mindesten 50%, bevorzugt von mindestens 30%, besonders bevorzugt von mindestens 20% geschlossen wird.

Der Bereich mit einem vergrößerten Strömungswiderstand kann grundsätzlich durch sehr unterschiedliche strukturelle Merkmale in allen möglichen Varianten bereitgestellt werden, solange er derart beschaffen ist, dass der Strömungswiderstand unter Einwirkung von Hitze reduziert wird. Darunter wird im Kontext der vorliegenden Erfindung verstanden, dass der Bereich mit einem vergrößerten Strömungswiderstand während einer gewissen Mindestdauer einer gewissen Mindesttemperatur ausgesetzt wird. Die Mindestdauer beträgt bevorzugt 10 Sek., stärker bevorzugt 5 Sek., noch stärker bevorzugt 3 Sek. und besonders bevorzugt 2 Sek. Die Mindesttemperatur beträgt bevorzugt 140°C, stärker bevorzugt 120°C und besonders bevorzugt 100°C. Dementsprechend ist es besonders bevorzugt, dass der Bereich mit einem vergrößerten Strömungswiderstand derart beschaffen ist, dass der Strömungswiderstand reduziert wird, wenn der Bereich mindestens 2 Sekunden lang einer Temperatur von mindestens 100°C ausgesetzt wird.

Dabei soll der Strömungswiderstand des Bereichs unter Einwirkung von Hitze soweit reduziert werden, dass die dadurch verursachte Zunahme des Volumenstroms bzw. der dadurch verursachte Druckabfall von einem Detektor bzw. Sensor erfasst werden kann und/oder direkt zu einem Schließen des Ventils führt. Dementsprechend hängt die erfindungsgemäß notwendige Reduzierung des Strömungswiderstands von der Art des verwendeten Detektors bzw. von der Empfindlichkeit des Ventils ab sowie von den Bedingungen (Druck und Volumenstrom) ab, unter denen das Atemgas verabreicht wird.

Letztere hängen unter anderem vom Patienten (manche Patienten werden mit einem Volumenstrom von z.B. 5 l/min, andere mit einem Volumenstrom von z.B. 3 l/min versorgt) und von der Art der Einrichtung zur Bereitstellung des angereicherten Atemgases ab.

Es ist jedoch bevorzugt, dass der Bereich mit einem vergrößerten Strömungswiderstand einen Strömungswiderstand aufweist, der einer Blende mit einem lichten Durchmesser zwischen 0,1 mm und 4 mm, stärker bevorzugt zwischen 0,3 mm und 3 mm und besonders bevorzugt zwischen 0,5 mm und 2 mm entspricht. Ferner ist bevorzugt, dass der Bereich mit einem vergrößerten Strömungswiderstand einen Strömungswiderstand aufweist, der kleiner oder gleich dem Strömungswiederstand einer Blende mit einem lichten Durchmesser mit 2 mm, bevorzugt mit 1,5 mm und besonders bevorzugt mit 1 mm ist. Es ist ferner bevorzugt, dass, beispielsweise im Falle mehrerer Öffnungen mit reduzierter Querschnittsfläche, die größte Öffnung in dem Bereich mit einem vergrößerten Strömungswiderstand eine Querschnittsfläche von maximal 10 mm², bevorzugt von maximal 5 mm², stärker bevorzugt von maximal 3 mm², noch stärker bevorzugt von maximal 2 mm² und besonders bevorzugt von maximal 1 mm² aufweist. Ferner ist bevorzugt, dass der Bereich mit einem vergrößerten Strömungswiderstand eine Querschnittsfläche aufweist, die maximal 40%, bevorzugt maximal 30%, stärker bevorzugt maximal 20%, noch stärker bevorzugt maximal 15% und besonders bevorzugt maximal 10% der maximalen Querschnittsfläche der restlichen Zuführleitung beträgt. Es ist ferner bevorzugt, dass der Bereich mit einem vergrößerten Strömungswiderstand einen Strömungswiderstand aufweist, der gegenüber dem Strömungswiderstand der restlichen Zuführleitung bei einem Volumenstrom von 5 l/min um mindestens 10%, bevorzugt um mindestens 20%, stärker bevorzugt um mindestens 30%, noch stärker bevorzugt um mindestens 50% und besonders bevorzugt um mindestens 100% erhöht ist. Je nachdem, ob eine laminare oder turbulente Strömung vorliegt, kann der Strömungswiderstand auch um einen Faktor von mindestens 2, bevorzugt mindestens 3, stärker bevorzugt mindestens 5 und besonders bevorzugt mindestens 8 erhöht werden. Es ist ferner bevorzugt, dass der Bereich mit einem vergrößerten Strömungswiderstand derart beschaffen ist, dass eine Entfernung und/oder Zerstörung des Bereichs bei einem Volumenstrom von 5 l/min zu einem Druckabfall in der Zuführleitung von mindestens 0,05 bar, stärker bevorzugt von mindestens 0,1 bar und besonders bevorzugt von mindestens 0,2 bar führt.

Derartige Bereiche mit einem vergrößerten Strömungswiderstand können auf unterschiedliche Art und Weise realisiert werden. Beispielsweise kann der Bereich mit einem vergrößerten Strömungswiderstand eines oder einer Kombination der folgenden Elemente aufweisen: Bereich mit reduziertem Öffnungsquerschnitt, Blende, Düse, mehrere Öffnungen mit reduzierter Querschnittsfläche, labyrinthartige Struktur, Sieb, Filter wie z.B. Sinterfilter, Tiefenfilter aus Zellulose oder Kunststofffasern oder Membranfilter.

Die Reduktion des Strömungswiderstands unter Einwirkung von Hitze kann dabei mittels unterschiedlicher Wirkmechanismen erzielt werden. Beispielsweise kann der Bereich aus einem Material bestehen, das unter Einwirkung von Hitze seine Stabilität verliert und/oder schmilzt. So kann der Bereich mit einem erhöhten Strömungswiderstand beispielsweise aus Polyethylen mit einer Schmelztemperatur von ca. 100°C gefertigt sein. Unter Einwirkung von Hitze schmilzt dann beispielsweise der Bereich mit erhöhtem Öffnungsquerschnitt, die Düse, etc., sodass der Strömungswiderstand in dem Bereich reduziert wird und bei vollständigem Schmelzen dem Strömungswiderstand in der restlichen Zuführleitung entspricht. Alternativ könnte die Reduktion des Strömungswiderstands unter Einwirkung von Hitze auch durch ein leicht brennbares Material bewirkt werden. So könnte beispielsweise ein Filter aus Zellulose in der Zuführleitung verbrennen und so den Strömungswiderstand reduzieren.

Das Ventil ist bevorzugt in oder an der Einrichtung zur Bereitstellung von sauerstoffangereichertem Atemgas oder in der Zuleitung vorgesehen.

Bevorzugt ist die Vorrichtung ferner dazu geeignet, einen Alarm auszulösen, wenn das Ventil die Zufuhr des sauerstoffangereicherten Atemgases unterbindet. Dieser Alarm kann beispielsweise durch die Steuerung ausgelöst werden, die das Ventil schließt. Alternativ könnte mit der Ventilauslösung beispielsweise ein elektrischer Kontakt betätigt werden, der zur Alarmierung durch einen Gerätealarm oder einen externen Alarm führt.

Die Vorrichtung weist bevorzugt ferner eine Quelle für nichtbrennbares Gas, insbesondere Stickstoff, Edelgas und/oder Kohlendioxid, auf, wobei die Vorrichtung dazu geeignet ist, die Zuführleitung mit dem nichtbrennbaren Gas zu fluten, wenn das Ventil die Zufuhr des sauerstoffangereicherten Atemgases unterbindet. Hierfür kann beispielsweise ein Zweiwegeventil oder ein Bypass distal vom Ventil vorgesehen sein.

Wenn das erfindungsgemäße Ventil bzw. dessen Steuerung immer dann anspricht, wenn der Volumenstrom von sauerstoffangereichertem Atemgas zunimmt oder ein Druckabfall erfolgt, so kann es passieren, dass beispielsweise beim Anfahren des Konzentrators oder beim Verbinden der Zuleitung mit der Einrichtung zur Bereitstellung von sauerstoffangereichertem Atemgas ein schneller Druckanstieg erfolgt, der dazu führen könnte, dass das Ventil ungewollt geschlossen wird. Daher ist bevorzugt eine Einrichtung vorgesehen, die ein ungewolltes Schließen des Ventils verhindert. Beispielsweise könnte bei einem elektronisch gesteuerten Ventil dafür gesorgt werden, dass die Steuerung des Ventils erst mit einer Zeitverzögerung von beispielsweise wenigen Sekunden nach dem Anfahren des Konzentrators und/oder Verbindung der Zuleitung mit der Einrichtung zur Bereitstellung von sauerstoffangereichertem Atemgas eingeschaltet wird.

Nachdem sich die Steuerung eingeschaltet hat, misst sie zunächst den bestehenden Druck oder Flow bzw. Volumenstrom, um danach auf Druckabfall oder Flowzunahme (bzw. Volumenstromzunahme) reagieren zu können.

Im Falle eines Ventils, das dazu geeignet ist, mechanisch selbsttätig die Zufuhr des sauerstoffangereicherten Atemgases zu unterbinden, kann das Ventil bevorzugt manuell in der Offenstellung fixiert werden. Beispielweise könnte das Ventilgehäuse zwei elastische Bereiche aufweisen, auf die der Anwender mit zwei Fingern drücken kann und die durch das Vorquellen des Elastomers in das Innere des Ventilgehäuses den Schließkörper des Ventils festhalten bzw. fixieren und nach dem Loslassen wieder freigeben. So kann der Anwender das Ventil in der Offenstellung fixieren, während er den Konzentrator startet, und anschließend das Ventil wieder freigeben. Wenn die Offenhaltung des Ventils magnetisch erfolgt, könnte der Schließkörper des Ventils auch berührungslos durch einen verschieblichen Eisenring in der Offenstellung gehalten werden.

Bei dem Ventil kann es sich beispielsweise um ein mechanisches Flow-Stopp-Ventil handeln, das beispielsweise als Einmalprodukt in das patientenferne Ende der Zuführleitung integriert ist. Alternativ könnte das Flow-Stopp-Ventil als separates, wiederverwendbares Teil direkt an die Einrichtung zur Bereitstellung von sauerstoffangereichertem Atemgas angeschlossen oder in diese integriert sein. Das Ventil kann auch verstellbar sein, um die Auslöseschwelle an den eingestellten Volumenstrom anzupassen. Diese Anpassung kann auch automatisch erfolgen. Wird das Ventil durch eine Steuerung gesteuert, so kann es sich bei dem Ventil beispielsweise um ein elektromagnetisches oder elektromotorisches Ventil handeln.

Die vorliegende Erfindung betrifft ferner ein Verfahren zum Betrieb eine Vorrichtung wie oben beschrieben. Das Verfahren weist die folgenden Schritte auf:
Aktivierung der Bereitstellung von sauerstoffangereichertem Atemgas durch Einschalten der Einrichtung zur Bereitstellung von sauerstoffangereichertem Atemgas und/oder Öffnen eines Ventils,
Messen des Volumenstroms von sauerstoffangereichertem Atemgas und/oder des Druck des sauerstoffangereicherten Atemgases und Definieren eines Schwellwerts auf Basis der Messung, wobei das Messen nach einer vorbestimmten Zeitspanne nach der Aktivierung erfolgt,
kontinuierliches oder wiederholtes Messen des Volumenstroms von sauerstoffangereichertem Atemgas und/oder des Druck des sauerstoffangereicherten Atemgases, und

Deaktivierung der Bereitstellung von sauerstoffangereichertem Atemgas durch mechanisch selbsttätiges Schließen des Ventils, wenn eine prozentuale Mindestabweichung des gemessenen Volumenstroms von sauerstoffangereichertem Atemgas und/oder des gemessenen Drucks des sauerstoffangereicherten Atemgases vom Schwellwert ermittelt wird.

Im Kontext des erfindungsgemäßen Verfahrens können alle oben im Kontext mit der erfindungsgemäßen Vorrichtung beschriebenen bevorzugten Merkmale zum Einsatz kommen. Insbesondere beträgt die prozentuale Mindestabweichung bevorzugt 50%, stärker bevorzugt 30%, noch stärker bevorzugt 20% und besonders bevorzugt 10%. Die vorbestimmte Zeitspanne beträgt bevorzugt mindestens 10 s, stärker bevorzugt mindestens 20 s, noch stärker bevorzugt mindestens 30 s und besonders bevorzugt mindestens 1 min.

Nachfolgend werden bevorzugte Ausführungsformen der vorliegenden Erfindung unter Bezugnahme auf die Figuren näher beschrieben. Es zeigen:
Fig. 1 in perspektivischer Ansicht eine Zuführleitung mit Nasenbrille;
Fig. 2 in perspektivischer Ansicht die Nasenbrille gemäß Fig. 1;
Fig. 3 eine Längsschnittansicht durch eine erfindungsgemäße Nasenbrille gemäß einer bevorzugten Ausführungsform;
Fig. 4 eine Längsschnittansicht durch eine erfindungsgemäße Nasenbrille gemäß einer bevorzugten Ausführungsform;
Fig. 5 eine Längsschnittansicht durch eine erfindungsgemäße Nasenbrille gemäß einer bevorzugten Ausführungsform;
Fig. 6 eine Längsschnittansicht durch eine Nasenbrille gemäß einer bevorzugten Ausführungsform;
Fig. 7 eine Längsschnittansicht durch ein erfindungsgemäßes Ventil gemäß einer bevorzugten Ausführungsform;
Fig. 8 eine Längsschnittansicht durch ein erfindungsgemäßes Ventil gemäß einer bevorzugten Ausführungsform; und
Fig. 9 eine Längsschnittansicht durch ein erfindungsgemäßes Ventil gemäß einer bevorzugten Ausführungsform.

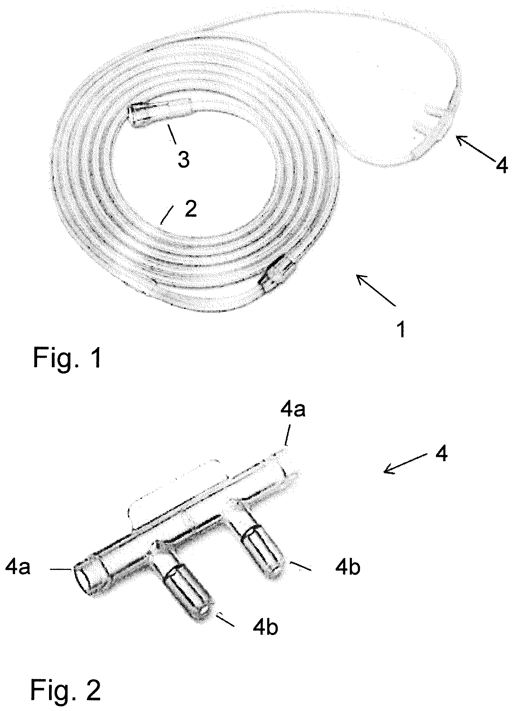

Figur 1 zeigt in perspektivischer Ansicht eine Zuführleitung 1, über die das sauerstoffangereicherte Atemgas von einer Einrichtung zur Bereitstellung von sauerstoffangereichertem Atemgas (nicht dargestellt) einem Patienten zugeführt werden kann. Die Zuführleitung 1 weist einen Zuführschlauch 2 mit einer Nasenbrille 4 und einem Konnektor 3 auf, mithilfe dessen die Zuführleitung mit der Einrichtung zur Bereitstellung von sauerstoffangereichertem Atemgas verbunden werden kann. Solche Einrichtungen zur Bereitstellung von sauerstoffangereichertem Atemgas sind aus dem Stand der Technik bekannt und bedürfen keiner näheren Beschreibung.

Figur 2 zeigt die Nasenbrille 4 gemäß Figur 1 nochmals in einer vergrößerten perspektivischen Ansicht. Die Nasenbrille 4 weist zwei Anschlussstücke 4a auf, mittels derer die Nasenbrille 4 mit dem Zuführschlauch 2 verbunden werden kann. Ferner weist die Nasenbrille 4 zwei Nasenstutzen 4b auf, die in die Nasenlöcher eines Patienten eingeführt werden können.

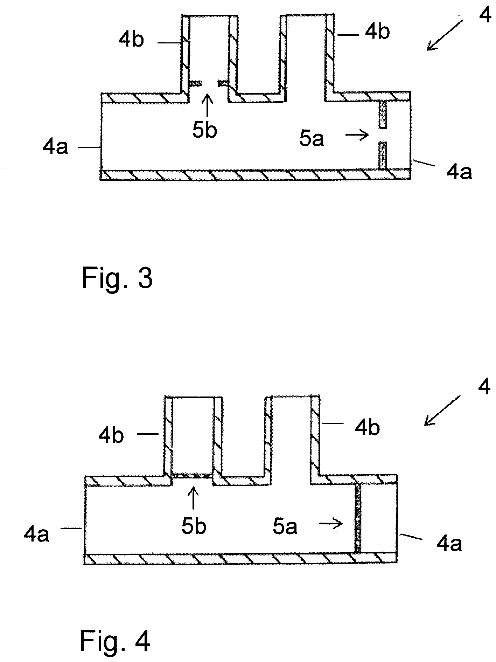

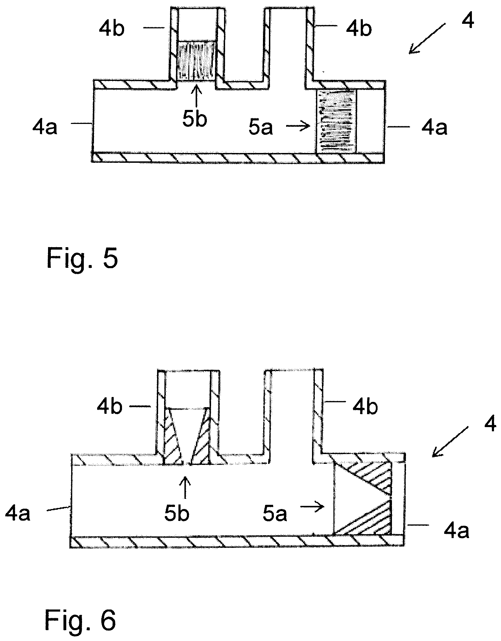

In den Figuren 3 bis 6 sind jeweils in Längsschnittansicht bevorzugte Ausführungsformen einer erfindungsgemäßen Nasenbrille schematisch dargestellt. Die in den Figuren 3 bis 6 gezeigten Nasenbrillen 4 weisen ebenfalls jeweils zwei Anschlussstücke 4a und zwei Nasenstutzen 4b auf. Ferner sind in den Nasenbrillen 4 jeweils zwei Bereiche mit einem vergrößerten Strömungswiderstand 5a und 5b dargestellt. Dabei ist jeweils ein solcher Bereich 5a in einem der Anschlussstücke 4a gezeigt und einer der Bereiche 5b in einem der Nasenstutzen 4b gezeigt. Die durch die Bezugszeichen 5a und 5b gekennzeichneten Bereiche stellen dabei alternative Ausführungsvarianten dar. In den tatsächlichen Ausführungsformen der Nasenbrille 4 ist bevorzugt in jedem der Anschlussstücke 4a ein Bereich 5a mit vergrößertem Strömungswiderstand vorgesehen oder alternativ in jedem der Nasenstutzen 4b ein Bereich 5b mit vergrößertem Strömungswiderstand vorgesehen. Der Bereich mit einem vergrößertem Strömungswiderstand kann dabei beispielsweise durch einen Bereich mit reduziertem Öffnungsquerschnitt wie z.B. eine Blende (vgl. Figur 3), durch ein Sieb oder einen Membranfilter (vgl. Figur 4), durch einen porösen Tiefenfilter aus Sintermaterial oder Fasermaterial (vgl. Figur 5) oder durch eine Düse (vgl. Figur 6) bereitgestellt werden.

Eine einfache Blende, wie sie in Figur 3 dargestellt ist, kann unter Umständen zu einem Austritt von sauerstoffangereichertem Atemgas in Form eines scharfen oder fokussierten Strahls führen, was in der Nase des Patienten unangenehme Empfindungen auslösen kann. Es ist daher bevorzugt, den Bereich mit vergrößertem Strömungswiderstand derart auszubilden, dass es nicht zu einer Erhöhung der Austrittsgeschwindigkeit des Atemgases kommt. Dies kann beispielsweise durch viele kleine Öffnungen (vgl. Figur 4) anstelle einer großen Durchtrittsöffnung bewerkstelligt werden. Mithilfe von Kunststoffspritzguss kann auch eine labyrinthartige Struktur erzeugt werden, die zu einer entsprechenden Abbremsung des Atemgasstroms führt. Auch Filter, beispielsweise Membranfilter oder Tiefenfilter aus Zellulose oder Kunststoff oder aus porösen Materialien wie z.B. gesintertem Polyethylen oder einer Glasfritte, haben einen ähnlichen Effekt. Ebenso kann ein Bereich mit erhöhtem Strömungswiderstand in Form einer Düse (vgl. Figur 6) zu einer Aufweitung des Atemgasstrahls führen.

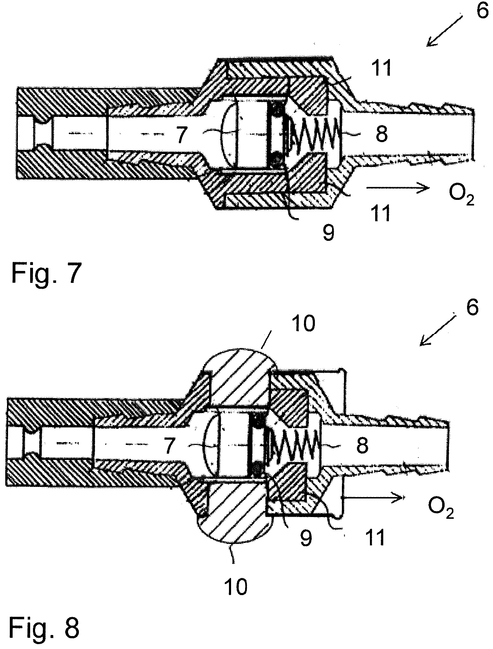

Figur 7 zeigt in einer Längsschnittansicht ein erfindungsgemäßes Ventil gemäß einer bevorzugten Ausführungsform. Bei dem Ventil 6 handelt es sich um ein Ventil, das dazu geeignet ist, mechanisch selbsttätigt die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases zu unterbinden. Das Ventil 6 weist einen Verschlusskörper 7 auf, der im in Figur 7 dargestellten Zustand aufgrund des mittels eines Pfeils illustrierten Stroms von Sauerstoff, wenn dieser beispielsweise plötzlich zunimmt, nach rechts mit seiner Dichtung 9 gegen den Ventilsitz 11 gedrückt wird, sodass das Ventil 6 im in Figur 7 gezeigten Zustand geöffnet ist. Bei einem kleineren Volumenstrom hingegen hält die Feder 8 den Verschlusskörper 7 entgegen der Stromrichtung des sauerstoffangereicherten Atemgases nach links in der geschlossenen Stellung. Die in Figur 7 gezeigte Offenstellung stellt gewissermaßen den Normalzustand des Ventils bei regulärer Zufuhrleistung von sauerstoffangereichertem Atemgas dar, wohingegen die geschlossene Stellung des Ventils 6 dann realisiert wird, wenn beispielsweise bei einem Volumenstrom von 5 l/min eine Zunahme des Volumenstroms um mindestens 30%, bevorzugt um mindestens 20% und besonders bevorzugt um mindestens 10% erfolgt. Eine solche Zunahme des Volumenstroms wird erfindungsgemäß dadurch verursacht, dass der Strömungswiderstand des Bereichs mit einem vergrößerten Strömungswiderstand unter Einwirkung von Hitze reduziert wird. Beispielsweise können die in den Figuren 3 bis 6 dargestellten Strukturen der jeweiligen Bereiche 5a und 5b unter Einwirkung von Hitze abschmelzen oder soweit deformiert werden, dass ein messbarer Anstieg des Volumenstroms und/oder ein messbarer Druckabfall verursacht wird.

Um zu verhindern, dass das Ventil beispielsweise beim Einschalten des Konzentrators oder beim Verbinden der Zuleitung mit dem Konzentrator wegen eines dadurch verursachten Druckanstiegs ungewollt selbsttätig schließt, kann eine Einrichtung am Ventil 6 vorgesehen sein, die es ermöglicht, den Verschlusskörper 7 in der Offenstellung zu fixieren. Beispielsweise können, wie dies schematisch in Figur 8 dargestellt ist, zwei elastische Bereiche 10 (oder eine ringförmige Struktur aus einem Elastomer) vorgesehen sein, auf die der Anwender mit zwei Fingern drücken kann, was ein Vorquellen des Elastomers 10 in das Innere des Ventilgehäuses bewirkt, wodurch der Schließkörper 7 des Ventils 6 in seiner aktuellen Position gehalten wird.

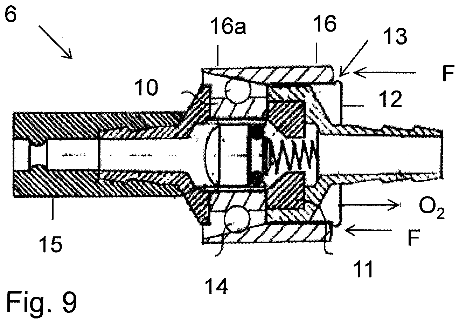

Zusätzlich oder alternativ kann ein verschiebbar angeordneter Ring 16 vorgesehen sein, der sich um einen Ventilgehäuseabschnitt 12 herum erstreckt und in distaler und proximaler Richtung über den Ventilgehäuseabschnitt 12 gleiten kann, wie dies in Figur 9 dargestellt ist. Am distalen Ende des Ventilgehäuseabschnitts 12 kann ein Vorsprung 13 oder eine andere Einrichtung zur Begrenzung des Verschiebewegs des Rings 16 in distaler Richtung vorgesehen sein. Wird nun das proximale Anschlussstück 15 des Ventils 6 mit z.B. einem Konzentrator verbunden, so wird der Anwender, wenn er das Ventil 6 am Ring 16 greift, eine in proximaler Richtung wirkende Kraft (vgl. Pfeil) auf den Ring 16 ausüben, die wegen des keilförmigen proximalen Abschnitts 16a des Rings 16 dazu führt, dass die Kugeln 14 in das Elastomer 10 gedrückt werden, so dass ein Vorquellen des Elastomers 10 in das Innere des Ventilgehäuses den Schließkörper 7 des Ventils 6 in seiner aktuellen Position hält.

Anstelle der Feder 8 kann das Ventil 6 auch einen magnetischen Mechanismus aufweisen, mithilfe dessen das Ventil entgegen der Stromrichtung des sauerstoffangereicherten Atemgases in die Offenstellung vorgespannt wird. Ferner kann das Ventil auch ein elektromagnetisches oder elektromotorisches Ventil sein, das mittels einer Kombination aus Sensor und Steuerung kontrolliert wird.

## Patentansprüche

1. Vorrichtung zur Versorgung eines Patienten mit sauerstoffangereichertem Atemgas, mit:
einer Einrichtung zur Bereitstellung von sauerstoffangereichertem Atemgas;
einer mit der Einrichtung verbundenen Zuführleitung (1), über die das sauerstoffangereicherte Atemgas von der Einrichtung einem Patienten zugeführt werden kann; und
einem Detektor, der dazu geeignet ist, eine Zunahme des Volumenstroms von sauerstoffangereichertem Atemgas und/oder einen Druckabfall des sauerstoffangereicherten Atemgases zu detektieren;
wobei die Vorrichtung dazu geeignet ist, die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases zu unterbinden, und
wobei die Zuführleitung (1) einen patientennahen Bereich (5a; 5b) mit einem gegenüber dem Strömungswiderstand der restlichen Zuführleitung vergrößerten Strömungswiderstand aufweist, der derart beschaffen ist, dass der Strömungswiderstand unter Einwirkung von Hitze reduziert wird;
ferner mit einem Ventil (6), das die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases unterbindet, wobei das Ventil (6) dazu geeignet ist, mechanisch selbsttätig die Zufuhr des sauerstoffangereicherten Atemgases im Falle einer Zunahme des Volumenstroms von sauerstoffangereichertem Atemgas und/oder eines Druckabfalls des sauerstoffangereicherten Atemgases zu unterbinden.

2. Vorrichtung nach Anspruch 1, ferner mit einer Steuerung, die dazu geeignet ist, die Einrichtung zur Bereitstellung von sauerstoffangereichertem Atemgas abzuschalten und/oder ein Ventil zu schließen, falls der Detektor eine Zunahme des Volumenstroms von sauerstoffangereichertem Atemgas und/oder einen Druckabfall des sauerstoffangereicherten Atemgases detektiert, wobei die Steuerung bevorzugt dazu eingerichtet ist, im Normalbetrieb, bevorzugt nach einer vorbestimmten Zeitspanne nach dem Beginn der Bereitstellung von sauerstoffangereichertem Atemgas, mittels des Detektors den Volumenstrom von sauerstoffangereichertem Atemgas und/oder den Druck des sauerstoffangereicherten Atemgases zu messen und einen Schwellwert zu definieren.

3. Vorrichtung nach Anspruch 2, wobei die Steuerung dazu eingerichtet ist, die Zufuhr des sauerstoffangereicherten Atemgases zu unterbinden, wenn eine prozentuale Mindestabweichung des gemessenen Volumenstroms von sauerstoffangereichertem Atemgas und/oder des gemessenen Drucks des sauerstoffangereicherten Atemgases vom Schwellwert ermittelt wird, wobei bevorzugt die prozentuale Mindestabweichung 30%, stärker bevorzugt 20% und besonders bevorzugt 10% beträgt.

4. Vorrichtung nach einem der vorigen Ansprüche, wobei der Detektor dazu geeignet ist, eine relative Zunahme des Volumenstroms von sauerstoffangereichertem Atemgas um 30%, bevorzugt um 20%, besonders bevorzugt um 10% und/oder einen relativen Druckabfall des sauerstoffangereicherten Atemgases von 30%, bevorzugt von 20%, besonders bevorzugt von 10% zu detektieren.

5. Vorrichtung nach einem der vorigen Ansprüche, wobei das Ventil (6) in oder an der Einrichtung zur Bereitstellung von sauerstoffangereichertem Atemgas oder in der Zuleitung (1) vorgesehen ist, wobei das Ventil (6) bevorzugt eine Feder (8) oder einen Magneten aufweist, die/der das Ventil (6) entgegen der Stromrichtung des sauerstoffangereicherten Atemgases in die Offenstellung vorspannt, wobei die Federkonstante der Feder (8) oder die Kraft des Magneten bevorzugt derart bemessen ist, dass das Ventil (6) bei einem Volumenstrom von 5 l/min und einem Druckabfall von mindestens 30%, bevorzugt von mindestens 20%, besonders bevorzugt von mindestens 10% geschlossen wird.

6. Vorrichtung nach einem der vorigen Ansprüche, wobei das Ventil (6) einen Verschlusskörper (7) und eine Einrichtung aufweist, die dazu geeignet ist, den Verschlusskörper manuell in der Offenstellung des Ventils zu fixieren.

7. Vorrichtung nach einem der vorigen Ansprüche, wobei der Bereich (5a; 5b) mit einem vergrößerten Strömungswiderstand einen Strömungswiderstand aufweist, der einer Blende mit einem lichten Durchmesser zwischen 0,1 mm und 4 mm, bevorzugt zwischen 0,3 mm und 3 mm und besonders bevorzugt zwischen 0,5 mm und 2 mm entspricht und/oder der kleiner oder gleich dem Strömungswiederstand einer Blende mit einem lichten Durchmesser mit 2 mm, bevorzugt mit 1,5 mm und besonders bevorzugt mit 1 mm ist.

8. Vorrichtung nach einem der vorigen Ansprüche, wobei die größte Öffnung in dem Bereich (5a; 5b) mit einem vergrößerten Strömungswiderstand eine Querschnittsfläche von maximal 10 mm², bevorzugt von maximal 5 mm², stärker bevorzugt von maximal 3 mm², noch stärker bevorzugt von maximal 2 mm² und besonders bevorzugt von maximal 1 mm² aufweist und/oder wobei der Bereich (5a; 5b) mit einem vergrößerten Strömungswiderstand eine Querschnittsfläche aufweist, die maximal 40%, bevorzugt maximal 30%, stärker bevorzugt maximal 20%, noch stärker bevorzugt maximal 15% und besonders bevorzugt maximal 10% der maximalen Querschnittsfläche der restlichen Zuführleitung beträgt.

9. Vorrichtung nach einem der vorigen Ansprüche, wobei der Bereich (5a; 5b) mit einem vergrößerten Strömungswiderstand einen Strömungswiderstand aufweist, der gegenüber dem Strömungswiderstand der restlichen Zuführleitung bei einem Volumenstrom von 5 l/min um mindestens 10%, bevorzugt um mindestens 20%, stärker bevorzugt um mindestens 30%, noch stärker bevorzugt um mindestens 50% und besonders bevorzugt um mindestens 100% erhöht ist.

10. Vorrichtung nach einem der vorigen Ansprüche, wobei der Bereich (5a; 5b) mit einem vergrößerten Strömungswiderstand derart beschaffen ist, dass eine Entfernung und/oder Zerstörung des Bereichs bei einem Volumenstrom von 5 l/min zu einem Druckabfall in der Zuführleitung von mindestens 0,05 bar, bevorzugt von mindestens 0,1 bar und besonders bevorzugt von mindestens 0,2 bar führt.

11. Vorrichtung nach einem der vorigen Ansprüche, wobei der Bereich (5a; 5b) mit einem vergrößerten Strömungswiderstand eines oder eine Kombination der folgenden Elemente aufweist: Bereich mit reduziertem Öffnungsquerschnitt, Blende, Düse, mehrere Öffnungen mit reduzierter Querschnittsfläche, labyrinthartige Struktur, Sieb, Filter wie z.B. Sinterfilter, Tiefenfilter aus Zellulose oder Kunststofffasern oder Membranfilter.

12. Vorrichtung nach einem der vorigen Ansprüche, wobei die Zuführleitung (1) einen Zuführschlauch (2) mit einer Nasenbrille (4) oder einem Konnektor zur Verbindung mit einer Atemmaske oder einem Beatmungstubus aufweist, wobei der Bereich (5a; 5b) mit einem vergrößerten Strömungswiderstand am patientenseitigen Ende des Zuführschlauchs (1), an der Nasenbrille (4) und/oder am Konnektor vorgesehen ist.

13. Vorrichtung nach einem der vorigen Ansprüche, ferner mit einer Quelle für nicht brennbares Gas, insbesondere Stickstoff, Edelgas und/oder Kohlendioxid, wobei die Vorrichtung dazu geeignet ist, die Zuführleitung mit dem nicht brennbaren Gas zu fluten, wenn die Zufuhr des sauerstoffangereicherten Atemgases unterbunden wird.

14. Verfahren zum Betrieb einer Vorrichtung nach einem der vorigen Ansprüche mit den folgenden Schritten:
Aktivierung der Bereitstellung von sauerstoffangereichertem Atemgas durch Einschalten der Einrichtung zur Bereitstellung von sauerstoffangereichertem Atemgas und/oder Öffnen eines Ventils,
Messen des Volumenstroms von sauerstoffangereichertem Atemgas und/oder des Drucks des sauerstoffangereicherten Atemgases und Definieren eines Schwellwerts auf Basis der Messung, wobei das Messen nach einer vorbestimmten Zeitspanne nach der Aktivierung erfolgt,
kontinuierliches oder wiederholtes Messen des Volumenstroms von sauerstoffangereichertem Atemgas und/oder des Drucks des sauerstoffangereicherten Atemgases, und
Deaktivierung der Bereitstellung von sauerstoffangereichertem Atemgas durch mechanisch selbsttätiges Schließen des Ventils, wenn eine prozentuale Mindestabweichung des gemessenen Volumenstroms von sauerstoffangereichertem Atemgas und/oder des gemessenen Drucks des sauerstoffangereicherten Atemgases vom Schwellwert ermittelt wird.

15. Verfahren nach Anspruch 14, wobei die prozentuale Mindestabweichung 30%, bevorzugt 20% und besonders bevorzugt 10% beträgt.

## Claims

1. A device for supplying a patient with oxygenated breathable gas having:
an apparatus for providing oxygenated breathable gas;
a supply line (1) connected to the apparatus, through which the oxygenated breathable gas can be supplied from the apparatus to a patient; and
a detector which is suitable to detect an increase in the volume flow of oxygenated breathable gas and/or a drop in pressure of the oxygenated breathable gas;
wherein the device is suitable to stop the supply of the oxygenated breathable gas if the volume flow of oxygenated breathable gas increases and/or the pressure of the oxygenated breathable gas drops, and
wherein the supply line (1) comprises a patient-proximate section (5a; 5b) having an increased flow resistance vis-à-vis the flow resistance of the remaining part of the supply line, which is configured such that the flow resistance is reduced under the effect of heat;
further with a valve (6) which stops the supply of the oxygenated breathable gas if the volume flow of oxygenated breathable gas increases and/or the pressure of the oxygenated breathable gas drops, wherein the valve (6) is suitable to automatically stop the supply of the oxygenated breathable gas if the volume flow of oxygenated breathable gas increases and/or the pressure of the oxygenated breathable gas drops.

2. The device according to claim 1, further with a controller which is suitable to switch off the apparatus for providing oxygenated breathable gas and/or to close a valve in case the detector detects an increase in volume flow of oxygenated breathable gas and/or a drop in pressure of the oxygenated breathable gas, wherein the controller is preferably configured to measure the volume flow of oxygenated breathable gas and/or the pressure of the oxygenated breathable gas and to define a threshold value, in normal operation, preferably after a predetermined time period after the supply of oxygenated breathable gas started, by means of the detector.

3. The device according to claim 2, wherein the controller is configured to stop the supply of oxygenated breathable gas when a percentage minimum deviation of the measured volume flow of oxygenated breathable gas and/or the measured pressure of the oxygenated breathable gas from the threshold value is detected, wherein preferably the percentage minimum deviation is 30%, more preferably 20% and particularly preferably 10%.

4. The device according to any one of the preceding claims, wherein the detector is suitable to detect a relative increase in the volume flow of oxygenated breathable gas by 30%, preferably by 20%, particularly preferably by 10% and/or a relative drop in the pressure of the oxygenated breathable gas by 30%, preferably by 20%, particularly preferably by 10%.

5. The device according to any one of the preceding claims, wherein the valve (6) is provided in or at the apparatus for providing oxygenated breathable gas or in the supply line (1), wherein the valve (6) preferably comprises a spring (8) or a magnet which prestresses the valve (6) opposite to the flow direction of the oxygenated breathable gas into the open position, wherein the spring constant of the spring (8) or the force of the magnet is set such that the valve (6) is closed at a volume flow of 5 l/min and a drop in pressure of at least 30%, preferably of at least 20%, particularly preferably of at least 10%.

6. The device according to any one of the preceding claims, wherein the valve (6) has a closing body (7) and a means which is suitable to manually secure the closing body in the open position of the valve.

7. The device according to any one of the preceding claims, wherein the section (5a; 5b) with an increased flow resistance has a flow resistance which corresponds to an aperture having an inner diameter between 0.1 mm and 4 mm, preferably between 0.3 mm and 3 mm and particularly preferably between 0.5 mm and 2 mm and/or is smaller than or equal to the flow resistance of an aperture having an inner diameter of 2 mm, preferably of 1.5 mm and particularly preferably of 1 mm.

8. The device according to any one of the preceding claims, wherein the largest opening in the section (5a; 5b) having an increased flow resistance comprises a cross-sectional area of 10 mm² at the most, preferably 5 mm² at the most, more preferably 3 mm² at the most, even more preferably 2 mm² at the most and particularly preferably 1 mm² at the most and/or wherein the section (5a; 5b) having an increased flow resistance comprises a cross-sectional area of 40% at the most, preferably 30% at the most, more preferably 20% at the most, even more preferably 15% at the most and particularly preferably 10% at the most of the maximum cross-sectional area of the remaining part of the supply line.

9. The device according to any one of the preceding claims, wherein the section (5a; 5b) having an increased flow resistance comprises a flow resistance which is increased by at least 10%, preferably by at least 20%, more preferably by at least 30%, even more preferably by at least 50% and particularly preferably by at least 100% vis-à-vis the flow resistance of the remaining part of the supply line at a volume flow of 5 l/min.

10. The device according to any one of the preceding claims, wherein the section (5a; 5b) having an increased flow resistance is configured such that a removal and/or destruction of the section at a volume flow of 5 l/min leads to a drop in pressure in the supply line of at least 0.05 bar, preferably at least 0.1 bar and particularly preferably of at least 0.2 bar.

11. The device according to any one of the preceding claims, wherein the section (5a; 5b) having an increased flow resistance comprises one or a combination of the following elements: section with reduced opening cross-section, aperture, nozzle, a plurality of openings with reduced cross-sectional area, labyrinth-like structure, sieve, filter such as, for example, sinter filters, depth filters made of cellulose or plastic fibres or membrane filters.

12. The device according to any one of the preceding claims, wherein the supply line (1) comprises a supply tube (2) with a nasal cannula (4) or a connector to connect to a breathing mask or a breathing tube, wherein the section (5a; 5b) with an increased flow resistance is provided at the patient-facing end of the supply tube (1), at the nasal cannula (4) and/or at the connector.

13. The device according to any one of the preceding claims, further with a source for incombustible gas, in particular nitrogen, noble gas and/or carbon dioxide, wherein the apparatus is suitable to flood the supply line with the incombustible gas when the supply of the oxygenated breathable gas is stopped.

14. A method for operating a device according to any one of the preceding claims comprising the following steps:
activation of the provision of oxygenated breathable gas by switching on the apparatus for supplying oxygenated breathable gas and/or opening of a valve,
measuring the volume flow of oxygenated breathable gas and/or the pressure of the oxygenated breathable gas and defining a threshold value on the basis of the measurement, wherein the measurement is conducted after a predetermined time period after the activation,
continuous or repeated measurement of the volume flow of oxygenated breathable gas and/or the pressure of the oxygenated breathable gas, and
deactivation of the supply of oxygenated breathable gas by automatic mechanical closing of the valve, when a percentage minimum deviation of the measured volume flow of oxygenated breathable gas and/or the measured pressure of the oxygenated breathable gas from the threshold is detected.

15. The method according to claim 14, wherein the percentage minimum deviation is 30%, preferably 20% and particularly preferably 10%.

## Revendications

1. Système pour l'alimentation d'un patient en gaz respiratoire enrichi en oxygène avec :
un dispositif de mise à disposition d'un gaz respiratoire enrichi en oxygène ;
une conduite d'alimentation (1) relié avec le dispositif, par l'intermédiaire de laquelle le gaz respiratoire enrichi en oxygène peut être introduit dans le patient par le dispositif ; et
un détecteur qui est conçu pour détecter une augmentation du débit volumique de gaz respiratoire enrichi en oxygène et/ou une chute de pression du gaz respiratoire enrichi en oxygène ;
dans lequel le système est conçu pour empêcher l'introduction du gaz respectivement enrichi en oxygène dans le cas d'une augmentation du débit volumique du gaz respiratoire enrichi en oxygène et/ou d'une chute de pression du gaz respiratoire enrichi en oxygène, et
dans lequel la conduite d'alimentation (1) comprend une partie proche du patient (5a ; 5b) avec une résistance à l'écoulement augmentée par rapport à la résistance à l'écoulement du reste de la conduite d'alimentation, qui est conçue de sorte que la résistance à l'écoulement est réduite sous l'effet de la chaleur ;
en outre avec une soupape (6) qui empêche l'introduction du gaz respiratoire enrichi en oxygène dans le cas d'une augmentation du débit volumique de gaz respiratoire enrichi en oxygène et/ou d'une chute de pression du gaz respiratoire enrichi en oxygène,
dans lequel la soupape (6) est conçue pour empêcher mécaniquement automatiquement l'introduction du gaz respiratoire enrichi en oxygène dans le cas d'une augmentation du débit volumique du gaz respiratoire enrichi en oxygène et/ou d'une chute de pression du gaz respiratoire enrichi en oxygène.

2. Système selon la revendication 1, en outre avec une commande qui est conçue pour arrêter le dispositif de mise à disposition en gaz respiratoire enrichi en oxygène et/ou pour fermer une soupape si le détecteur détecte une augmentation du débit volumique de gaz respiratoire enrichi en oxygène et/ou une chute de pression du gaz respiratoire enrichi en oxygène, dans lequel la commande est de préférence conçue, en fonctionnement normal, de préférence après un laps de temps prédéterminé après le début de la mise à disposition du gaz respiratoire enrichi en oxygène, pour mesurer, au moyen du détecteur, le débit volumique de gaz respiratoire enrichi en oxygène et/ou la pression du gaz respiratoire enrichi en oxygène et pour définir une valeur seuil.

3. Système selon la revendication 2, dans lequel la commande est conçue pour empêcher l'introduction du gaz respiratoire enrichi en oxygène lorsqu'un écart minimal en pourcentage entre le débit volumique mesuré du gaz respiratoire enrichi en oxygène et/ou la pression mesuré du gaz respiratoire enrichi en oxygène et la valeur seuil est déterminé, dans lequel, de préférence l'écart minimal en pourcentage est de 30 %; plus fortement de préférence de 20 % et plus particulièrement de préférence de 10 %.

4. Système selon l'une des revendications précédentes, dans lequel le détecteur est conçu pour détecter une augmentation relative du débit volumique de gaz respiratoire enrichi en oxygène de 30 %, de préférence de 20 %, plus particulièrement de préférence de 10 % et/ou une chute de pression relative du gaz respiratoire enrichi en oxygène de 30 %, de préférence de 20 %, plus particulièrement de préférence de 10 %.

5. Système selon l'une des revendications précédentes, dans lequel la soupape (6) est prévue dans ou sur le dispositif de mise à disposition d'un gaz respiratoire enrichi en oxygène ou dans la conduite d'alimentation (1), dans lequel la soupape (6) comprend de préférence un ressort (8) ou un aimant, qui précontraint la soupape (6) contre la direction d'écoulement du gaz respiratoire enrichi en oxygène dans la position ouverte, dans lequel la constante élastique du ressort (8) ou la force de l'aimant est conçue de manière à ce que la soupape (6) est fermée lors d'un débit volume de 5 l/min et une chute de pression d'au moins 30 %, de préférence d'au moins 20 %, plus particulièrement de préférence d'au moins 10 %.

6. Système selon l'une des revendications précédentes, dans lequel la soupape (6) comprend un corps de fermeture (7) et un dispositif qui est conçu pour fixer le corps de fermeture manuellement dans la position ouverte de la soupape.

7. Système selon l'une des revendications précédentes, dans lequel la partie (5a ; 5b) avec une résistance à l'écoulement augmentée présente une résistance à l'écoulement qui correspond à un diaphragme avec un diamètre intérieur entre 0,1 mm et 4 mm, de préférence entre 0,3 mm et 3 mm et plus particulièrement de préférence entre 0,5 mm et 2 mm et/ou qui est inférieure ou égal à la résistance à l'écoulement d'un diaphragme avec un diamètre intérieur de 2 mm, de préférence de 1,5 mm et plus particulièrement de préférence de 1 mm.

8. Système selon l'une des revendications précédentes, dans lequel l'ouverture la plus grande dans la partie (5a ; 5b) avec une résistance à l'écoulement augmentée présente une surface de section transversale de 10 mm² maximum, de préférence de 5 mm² maximum, plus fortement de préférence de 3 mm² maximum, encore plus fortement de préférence de 2 mm² maximum et plus particulièrement de préférence de 1 mm² maximum et/ou dans lequel la partie (5a ; 5b) avec une résistance à l'écoulement augmentée présente une surface de section transversale qui représente 40 % maximum, de préférence 30 % maximum, plus fortement de préférence 20 % maximum, encore plus fortement de préférence 15 % maximum et plus particulièrement de préférence 10 % maximum de la surface de section transversale maximale du reste de la conduite d'alimentation.

9. Système selon l'une des revendications précédentes, dans lequel la partie (5a ; 5b) avec une résistance à l'écoulement augmentée présente une résistance à l'écoulement qui est augmentée, par rapport à la résistance à l'écoulement du reste de la conduite d'alimentation, lors d'un débit volumique de 5 l/min, d'au moins 10 %, de préférence d'au moins 20 %, plus fortement de préférence d'au moins 30 %, encore plus fortement de préférence d'au moins 50 % et plus particulièrement de préférence d'au moins 100 %.

10. Système selon l'une des revendications précédentes, dans lequel la partie (5a ; 5b) avec une résistance à l'écoulement augmentée est conçue de sorte qu'une élimination et/ou une destruction de la partie conduit, lors d'un débit volumique de 5 l/min, à une chute de pression dans la conduite d'alimentation d'au moins 0,05 bar, de préférence d'au moins 0,1 bar et plus particulièrement de préférence d'au moins 0,2 bar.

11. Système selon l'une des revendications précédentes, dans lequel la partie (5a ; 5b) avec une résistance à l'écoulement augmentée comprend un des éléments ou une combinaison des éléments suivants : partie avec section transversale d'ouverture réduite, diaphragme, buse, plusieurs ouvertures avec surface de section transversale réduite, structure labyrinthique, tamis, filtre, par exemple filtre fritté, filtre profond en cellulose ou fibres de matière plastique ou filtre à membrane.

12. Système selon l'une des revendications précédentes, dans lequel la conduite d'alimentation (1) comprend un tuyau d'alimentation (2) avec une canule nasale (4) ou un connecteur pour le raccordement avec un masque respiratoire ou un tube de respiration, dans lequel la partie (5a ; 5b) avec une résistance à l'écoulement augmentée est prévue à l'extrémité côté patient du tuyau d'alimentation (1), sur la canule nasale (4) et/ou sur le connecteur.

13. Système selon l'une des revendications précédentes, en outre avec une source pour un gaz non combustible, plus particulièrement de l'azote, un gaz noble et/ou du dioxyde de carbone, dans lequel le système est conçu pour alimenter la conduite d'alimentation en gaz non combustible lorsque l'alimentation en gaz respiratoire enrichi en oxygène est inhibée.

14. Procédé de fonctionnement d'un système selon l'une des revendications précédentes avec les étapes suivantes :
activation de la mise à disposition d'un gaz respiration enrichi en oxygène par la mise en marche du dispositif de mise à disposition de gaz respiratoire enrichi en oxygène et/ou l'ouverture d'une soupape,
mesure du débit volumique de gaz respiratoire enrichi en oxygène et/ou de la pression du gaz respiratoire enrichi en oxygène et définition d'une valeur seuil sur la base de la mesure, dans lequel la mesure a lieu après un laps de temps prédéterminé après l'activation,
mesure en continu ou répétée du débit volumique de gaz respiratoire enrichi en oxygène et/ou de la pression du gaz respiratoire enrichi en oxygène, et
désactivation de la mise à disposition d'un gaz respiration enrichi en oxygène par la fermeture mécanique automatique de la soupape lorsqu'un écart minimal en pourcentage du débit volumique mesuré du gaz respiratoire enrichi en oxygène et/ou de la pression mesurée du gaz respiratoire enrichi en oxygène et la valeur seuil est déterminé.

15. Procédé selon la revendication 14, dans lequel l'écart minimal en pourcentage est de 30 %, de préférence de 20 % et plus particulièrement de préférence de 10 %.
